# EUROPEAN PATENT APPLICATION

(11) **EP 1 103 257 A2**
(43) Date of publication of application: **30.05.2001**
(21) Application number: 00310392.6
(22) Date of filing: 23.11.2000
(51) Int. Cl.: A61K 31/167, A61P 25/06

(54) **Method for treating migraine symptoms using acetominophen**

(30) Priority: 24.11.1999 US 448988; 06.11.2000 US 706681
(71) Applicant: McNEIL-PPC, INC., Skillman, New Jersey 08558 (US)
(72) Inventor: Codispoti, Joseph R, Philadelphia, PA 19116 (US)
(74) Representative: Mercer, Christopher Paul

(57) **Abstract**

The present invention provides a method for treating the photophobia and phonophobia symptoms of migraine attacks with an effective amount of acetaminophen, pharmaceutically acceptable salts thereof or mixtures thereof.

## Description

### Field of the Invention

The present invention relates to the treatment of migraine symptoms by the administration of an effective amount of acetaminophen, more specifically the present invention relates to the treatment of phonophobia and photophobia with acetaminophen, pharmaceutically acceptable salts thereof, or mixtures thereof.

### Background of the Invention

Migraine provides a wide variation of pain and symptoms, and associated disorders with those who suffer from the disease. Included among these symptoms are nausea, headache, moderate to severe pain, as well as incapacitating pain and total disability in a percentage of patients. Some patients also recite more specific symptoms such as photophobia, painful sensitivity to light; or phonophobia, painful sensitivity to sound.

Treatment of migraine pain includes both prescription and non-prescription or over-the-counter mediation. Among the over-counter medication that is currently available is a combination of acetaminophen/aspirin/caffeine, as set forth in US Patent 5,972,916, the contents hereby incorporated by reference as set forth herein in its entirety. The combination of active ingredients suffers from the drawback that some of the actives might cause undesired side effects. For example, it is well know that aspirin can cause stomach irritation and caffeine can cause anxiety and sleeplessness.

Therefore there is a continuing need for treatment of migraines, as well as treatment alternatives to specific migraine symptoms.

### Summary of the Invention

The present invention provides a method for treating photophobia and phonophobia associated with a migraine attack by providing an effective amount of acetaminophen, pharmaceutically acceptable salts thereof, or mixtures thereof. The present invention relies on the action of a single active ingredient, i.e., acetaminophen, pharmaceutically acceptable salts thereof, or mixtures thereof. The present invention also contemplates the use of additional ingredients, such as flavors, binders and excipients.

### Description of the Drawings

Figure 1 and Figure 3 depict the percentage of subjects with reduced photophobia over time after treatment with acetaminophen.

Figure 2 and Figure 4 depict the percentage of subjects with reduced phonophobia over time over time after treatment with acetaminophen.

### Detailed Description of the Invention

Acetaminophen is a well known analgesic material, which has analgesic and antipyrritic properties. It is commercially available in various forms for many years.

According to the present invention, acetaminophen, pharmaceutically acceptable salts thereof, or mixtures thereof, are preferably administered orally as an effective dose to alleviate the photophobia and phonophobia symptoms, which comprises from about 650 to about 1300 milligrams of acetaminophen per dose; preferably from 800 to about 1200 and most preferably from about 900 to about 1000 milligrams per dose. Typical doses of the pharmaceutically acceptable salts of acetaminophen will vary according to the molecular weight of the salt required to give the equivalent dose of acetaminophen. Typical oral doses of pharmaceutically acceptable salts of acetaminophen range from about 750 to about 3400 milligrams per dose. Typically doses are taken every four to six hours, but care should be taken to avoid the daily maximum recommended dosage of acetaminophen of 4000 milligrams per day.

Examples of suitable pharmaceutically acceptable salts of acetaminophen include inorganic salts of acetaminophen, including: sodium, calcium, lithium, potassium, magnesium, cesium, ammonia, ferrous, zinc, manganous, aluminum, ferric, manganic, and the like, organic salts of acetaminophen with primary, secondary, tertiary and quaternary amines, substituted amines including naturally occurring substituted amines, cyclic amines and basic ion exchange resins, triethylamine, tripropylamine, 2-dimethylaminoethanol, 2-diethylaminoethanol, lysine, arginine, histidine, caffeine, procain, N-ethylpiperidine, hydrabamine, choline, betaine, ethylenediamine, glucosamine, methylglycamine, theobromine, pruines, piperazine, piperidine, polyamine resins and the like. and mixtures thereof.

Suitable dosage forms include solids or liquids. Solid forms include tablets, capsules, liquid-filled soft gelatin capsules, powders, sachets and the like. Suitable liquids include suspensions, solutions, emulsions and the like.

The acetaminophen and/or pharmaceutically acceptable salts thereof may be admixed with various pharmaceutically-acceptable excipients and other ingredients including but not limited to diluents, granulating agents, disintegrating agents, binding agents, lubricants and the like. Examples of these various ingredients are set forth in US Patent 5,660,860, the contents hereby incorporated by reference as if set forth in its entirety.

The present invention as set forth in the following examples are meant to exemplify the various aspects of carrying out the present invention and are not intended to limit the invention in any way.

### Example 1

Subjects suffering from photophobia due to a migraine were either given a placebo or treated with 1000 milligrams of acetaminophen. One hundred seventy-five (175) subjects were treated with acetaminophen in tablet form, and one hundred fifty-nine (159) subjects were given placebos. Periodic assessments of their photophobia were made and the results are presented below.

**Table 1:**

| **Percentage (Number) of Subjects with Photo phobia Severity reduced to Zero by Time-Intent-to-Treat** | | | | | | | | |
|---|---|---|---|---|---|---|---|---|
| Treatment | 0.5* | 1* | 1.5* | 2* | 3* | 4* | 5* | 6* |
| APAP 1000 mg (N=175) | 2.3 (4) | 5.7 (10) | 13.7 (24) | 19.4 (34) | **31.4** **(55)** | 36.0 (63) | 39.4 (69) | 42.3 (74) |
| Placebo (N=159 | 3.1 (5) | 5.0 (8) | 11.9 (19) | 13.2 (21) | **14.5** **(23)** | 21.4 (34) | 25.8 (41) | 25.8 (41) |
| P value | | | | 0.127 | **0.004** | 0.005 | 0.004 | 0.002 |

| | | | | | | | | |
|---|---|---|---|---|---|---|---|---|
| * Assessment Timepoint (Hours) | | | | | | | | |

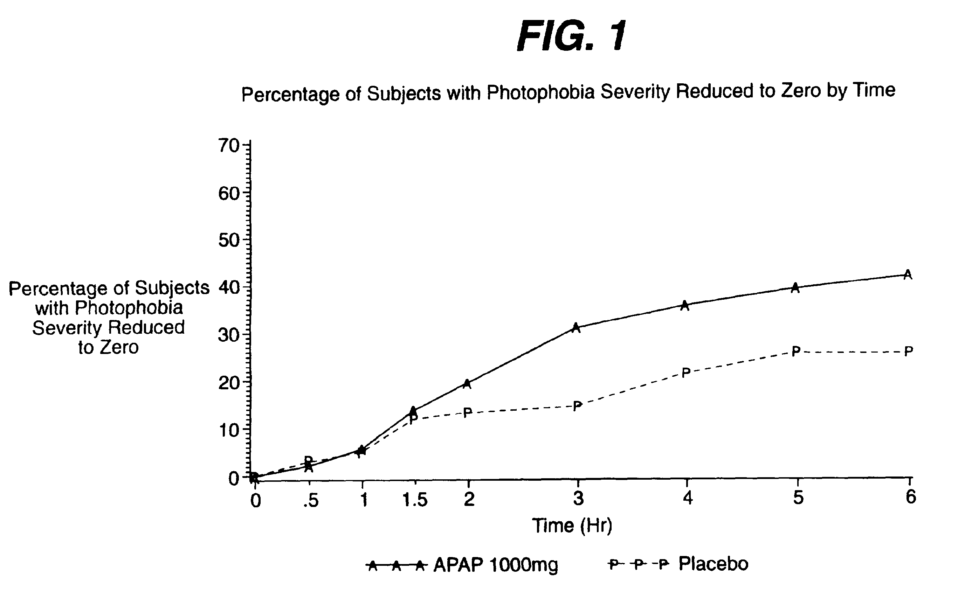

The results are also provided in Figure 1. The results indicate that at time of more than two hours or longer the treatment of photophobia with acetaminophen was effective in treating photophobia and statistically significant.

### Example 2

Subjects suffering from phonophobia due to a migraine were either given a placebo or treated with 1000 milligrams of acetaminophen in tablet form. One hundred sixty-two (162) subjects were treated with acetaminophen, and one hundred fifty-seven (157) subjects were given placebos. Periodic assessments of their phonophobia were made and the results are presented below.

**Table 2:**

| **Percentage (Number) of Subjects with Phonophobia Severity reduced to Zero by Time-Intent-to-Treat** | | | | | | | | |
|---|---|---|---|---|---|---|---|---|
| Treatment | 0.5* | 1* | 1.5* | 2* | 3* | 4* | 5* | 6* |
| APAP 1000 mg (N=162) | 3.1 (5) | 8.0 (13) | 16.0 (26) | **22.8** **(37)** | 30.9 (50) | 37.0 (60) | 40.7 (66) | 42.6 (69) |
| Placebo (N=157) | 3.2 (5) | 5.7 (9) | 10.2 (16) | **12.7** **(20)** | 17.2 (27) | 21.7 (34) | 25.5 (40) | 26.1 (41) |
| P value | | | | **0.018** | 0.018 | 0.002 | 0.001 | 0.002 |

| | | | | | | | | |
|---|---|---|---|---|---|---|---|---|
| * Assessment Timepoint (Hours) | | | | | | | | |

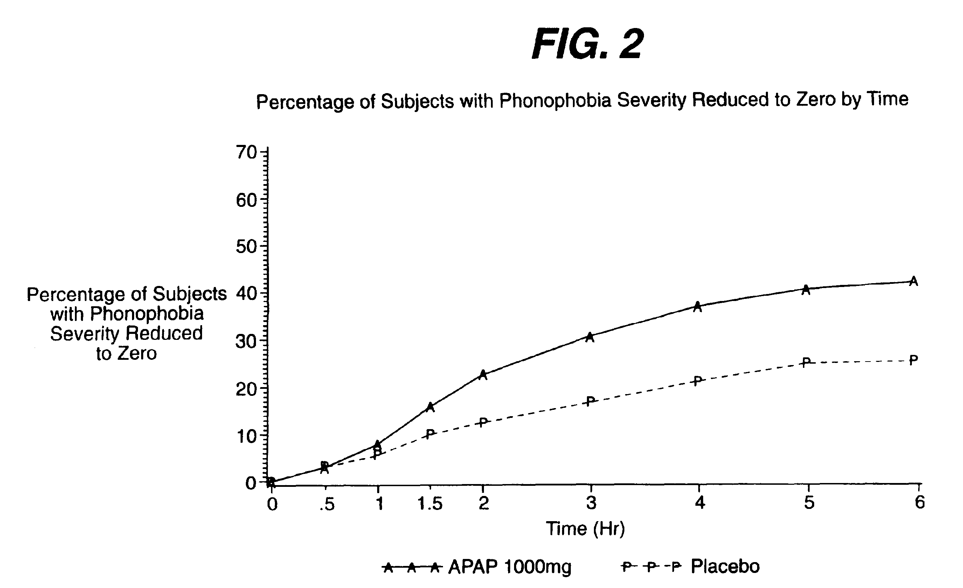

The results are also provided in Figure 2. The results indicate that at time of two hours or longer the treatment of phonophobia with acetaminophen was effective in treating phonophobia and statistically significant.

### Example 3

Subjects suffering from photophobia due to a migraine were either given a placebo or treated with 1000 milligrams of acetaminophen. One hundred forty-three (143) subjects were treated with acetaminophen in tablet form, and one hundred thirty-two (132) subjects were given placebos. Periodic assessments of their photophobia were made and the results are presented below.

**Table 3:**

| **Percentage (Number) of Subjects with Photophobia Severity reduced to Zero by Time-Intent-to-Treat** | | | | | | | | |
|---|---|---|---|---|---|---|---|---|
| Treatment | 0.5* | 1* | 1.5* | 2* | 3* | 4* | 5* | 6* |
| APAP 1000 mg (N=143) | 2.8 (4) | 14.0 (20) | 22.4 (32) | **32.9** **(47)** | 42.0 (60) | 46.2 (66) | 53.8 (77) | 58.7 (84) |
| Placebo (N=132) | 2.3 3) | 10.6 (14) | 15.9 (21) | **20.5** **(27)** | 25.0 (33) | 29.5 (39) | 31.8 (42) | 36.4 (48) |
| P value | | | | **0.024** | 0.001 | 0.003 | 0.008 | 0.001 |

| | | | | | | | | |
|---|---|---|---|---|---|---|---|---|
| * Assessment Timepoint (Hours) | | | | | | | | |

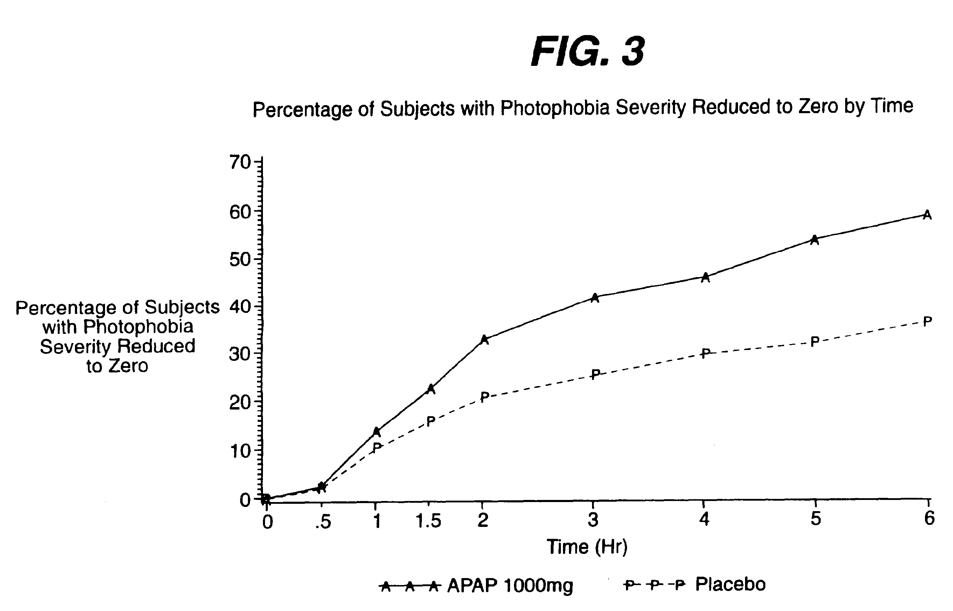

The results are also provided in Figure 3. The results indicate that at time of two hours or longer the treatment of photophobia with acetaminophen was effective in treating photophobia and statistically significant.

### Example 4

Subjects suffering from phonophobia due to a migraine were either given a placebo or treated with 1000 milligrams of acetaminophen in tablet form. One hundred thirty-nine (139) subjects were treated with acetaminophen, and one hundred twenty-nine (129) subjects were given placebos. Periodic assessments of their phonophobia were made and the results are presented below.

**Table 4:**

| **Percentage (Number) of Subjects with Phonophobia Severity reduced to Zero by Time-Intent-to-Treat** | | | | | | | | |
|---|---|---|---|---|---|---|---|---|
| Treatment | 0.5* | 1* | 1.5* | 2* | 3* | 4* | 5* | * |
| APAP 1000 mg (N=139) | 4.3 (6) | 13.7 (19) | 23.0 (32) | 33.8 (47) | **43.2** **(60)** | 48.9 (68) | 56.1 (78) | 61.2 (85) |
| Placebo (N=129) | 0.8 (1) | 8.5 (11) | 16.3 (21) | 23.3 (30) | **28.7** **(37)** | 30.2 (39) | 36.4 (47) | 40.3 (52) |
| P value | | | | 0.075 | **0.004** | 0.003 | 0.004 | 0.001 |

| | | | | | | | | |
|---|---|---|---|---|---|---|---|---|
| * Assessment Timepoint (Hours) | | | | | | | | |

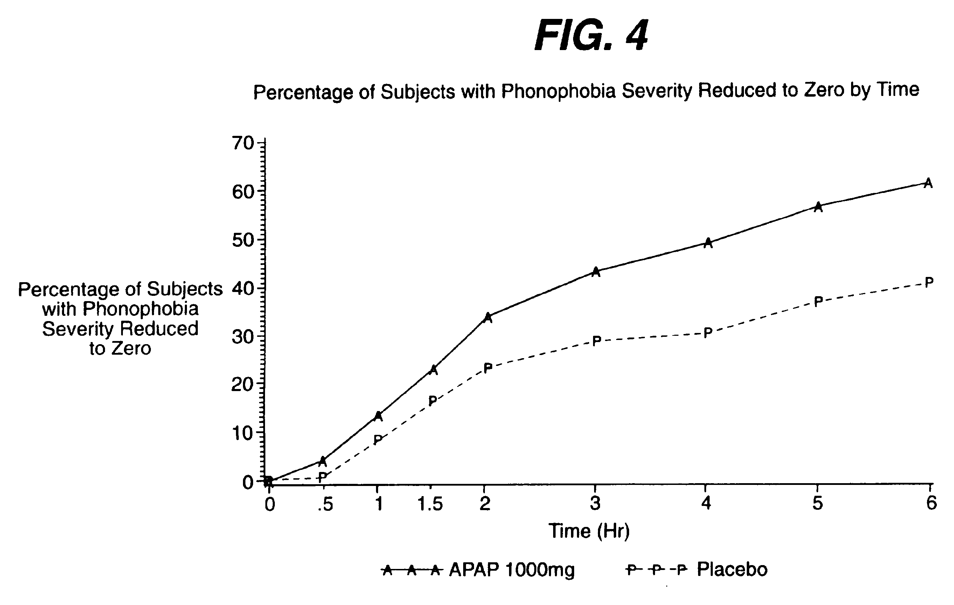

The results are also provided in Figure 4. The results indicate that at time of more than two hours or longer the treatment of phonophobia with acetaminophen was effective in treating phonophobia and statistically significant.

## Claims

1. A composition, for mitigating or treating photophobia or phonophobia associated with migraine, comprising an effective amount of acetaminophen, a pharmaceutically acceptable salt thereof of mixture thereof as the sole pharmaceutically effective ingredient.

2. The composition of claim 1 wherein the amount of acetaminophen is from 650 to 1300 milligrams per dosage, preferably about 1000 milligrams per dose.

3. The composition of claim 1 or claim 2 wherein the pharmaceutically acceptable salt of acetaminophen is
a) an inorganic salt of acetaminophen with sodium, calcium, lithium, potassium, magnesium, cesium, ammonium, ferrous, zinc, manganous, aluminum, ferric or manganic ion;
b) an organic salt of acetaminophen with a primary, secondary, tertiary or quaternary amine, a substituted amine such as a naturally occurring substituted amine, a cyclic amine, or a basic ion exchange resin, or
c) a mixture thereof.

4. The composition of claim 3 wherein the amine is triethylamine, tripropylamine, 2-dimethylaminoethanol, 2-diethylaminoethanol, lysine, arginine, histidine, caffeine, procain, N-ethylpiperidine, hydrabamine, choline, betaine, ethylenediamine, glucosamine, methylglycamine, theobromine, pruine, piperazine, piperidine, a polyamine resin or a mixture thereof.

5. The composition of claim 3 or claim 4 wherein the amount of the pharmaceutically acceptable salt of acetaminophen is from 750 to 3400 milligrams per dose.
